(19) **Europäisches Patentamt**
European Patent Office
Office européen des brevets

(11) **EP 1 699 498 B2**

(12) **NEW EUROPEAN PATENT SPECIFICATION**
After opposition procedure

(45) Date of publication and mention of the opposition decision:
**30.01.2013 Bulletin 2013/05**

(45) Mention of the grant of the patent:
**11.03.2009 Bulletin 2009/11**

(21) Application number: **04809061.7**

(22) Date of filing: **16.12.2004**

(51) Int Cl.:
**A61L 15/60** *(2006.01)*    **A61F 13/53** *(2006.01)*

(86) International application number:
**PCT/SE2004/001886**

(87) International publication number:
**WO 2005/063312 (14.07.2005 Gazette 2005/28)**

(54) **ABSORBENT STRUCTURE AND ABSORBENT ARTICLE COMPRISING SUPERABSORBENT PARTICLES**

SAUGFÄHIGE STRUKTUR UND SAUGFÄHIGER ARTIKEL ENTHALTEND SUPERABSORBIERENDE PARTIKEL

STRUCTURE ABSORBANTE ET ARTICLE ABSORBANT COMPRENANT DES PARTICULES SUPERABSORBANTES

(84) Designated Contracting States:
**AT BE BG CH CY CZ DE DK EE ES FI FR GB GR HU IE IS IT LI LT LU MC NL PL PT RO SE SI SK TR**

(30) Priority: **30.12.2003 SE 0303558**

(43) Date of publication of application:
**13.09.2006 Bulletin 2006/37**

(73) Proprietor: **SCA Hygiene Products Aktiebolag 405 03 Göteborg (SE)**

(72) Inventors:
• **ÖSTERDAHL, Eje**
  **S-425 58 Västra Frölunda (SE)**
• **HANSON, Charlotta**
  **S-412 55 Göteborg (SE)**

• **KARLSSON, Karl**
  **S-418 77 Göteborg (SE)**

(74) Representative: **Andersson, Per Rune et al Albihns.Zacco Torggatan 8 Box 142 S-401 22 Göteborg (SE)**

(56) References cited:
EP-A- 0 212 618          EP-A1- 0 339 461
EP-A2- 0 122 042         WO-A-2005/032443
WO-A1-97/12575           WO-A1-98/06364
WO-A1-2004/020008        GB-A- 2 272 459
US-A- 5 047 023          US-A- 5 061 259
US-A- 5 397 626          US-A- 5 462 972
US-A- 5 562 646          US-A- 5 714 156
US-B1- 6 380 456

**EP 1 699 498 B2**

**Description**

TECHNICAL FIELD

The present invention relates to an absorbent structure for use in an absorbent article such as a diaper, an incontinence pad, or a sanitary towel, which absorbent structure has at least one absorbent layer comprising fluff pulp and superabsorbent particles.

BACKGROUND ART

An absorbent structure for disposable absorbent articles such as diapers, incontinence pads and sanitary towels is usually constructed from one or more layers of hydrophilic fibres, for example cellulose fluff pulp. In order to obtain high absorption capacity and also a high liquid-retaining capacity when the article is subjected to external loading, the absorbent structure usually contains superabsorbent particles, which are polymers with the ability to absorb many times their own weight of water or body fluid. The effectiveness of the superabsorbent depends on many factors such as, for example, the physical shape of the superabsorbent particles. Other examples of properties which influence the functioning of the superabsorbent are absorption rate, gel strength and liquid-retaining capacity.

WO 2 005 032 443, constituting a prior art under Article 54(3) EPC, discloses an absorbent article such as a diaper, an incontinence pad, a sanitary towel having a liquid - permeable upper surface and comprising an absorbent structure which comprises a first end a second superabsorbent material. The first superabsorbent material is an odour-inhibiting and/or bacteria-inhibiting superabsorbent material and has a higher absorption rate than the second superabsorbent material.

The absorbent structure can also contain other components, for example in order to improve its liquid-spreading properties or increase its cohesive capacity and ability to withstand deformation during use.

It is of considerable importance that the absorbent article is capable of rapidly receiving and absorbing large quantities of liquid. It is also of considerable importance that the total absorption capacity of the article can be utilized. In order for it to be possible to utilize the total absorption capacity of the article, it is essential that the liquid can be spread from the wetting area to other parts of the absorbent structure.

One problem, above all for diapers and incontinence pads which are intended to receive and absorb relatively large quantities of liquid, is that there is a risk of them leaking before their total absorption capacity is fully utilized. One cause of leakage is that the absorbent structure, in particular when repeated wetting takes place, has an impaired ability to receive and absorb large quantities of liquid rapidly. A major cause of it being difficult for the absorbent structure to function satisfactorily when repeated wetting takes place, that is to say a second wetting and subsequent wettings, is that the superabsorbent material in a swollen state can block the pores in the porous fibrous structure and thus interfere with the transport of liquid from the wet area out to other parts of the absorbent structure. This phenomenon is referred to as gel blocking and results in the total absorption capacity of the absorbent structure not being utilized optimally. It also leads to an increased risk of leakage.

EP 0 212 618 A1 discloses an absorbent article including a layer of absorbent material which contains particles of superabsorbent material. Particles of superabsorbent material are distributed within the absorbent material layer, end these superabsorbent particles are disposed to form a substantially continuous, non-step-wise, positiv concentration gradient of superabsorbent through a portion of the thickness of the absorbent layer. The distinctive arrangement of the superabsorbent in a concentration gradient through the thickness of the absorbent layer reduces the occurrence of gel blocking end improves the reliability of the distribution of liquid throughout the superabsorbent material. The article, thus, provides for absorbent structures wherein the superabsorbent produced gel is adequately isolated away from body surface of a person using the article.

The problem of gel blocking increases when the proportion of superabsorbent material in an absorbent structure is high. In order to obtain an article which is discreet and comfortable to wear, however, it is an advantage to have a thin article which contains a relatively high proportion of superabsorbent material.

DISCLOSURE OF INVENTION

The problem of gel blocking during use of thin absorbent articles having a relatively high content of superabsorbent material has been reduced by means of the present invention.

An absorbent structure according to the invention is characterized as defined in claim 1. The absorption capacity is measured using 0.9% by weight sodium chloride solution.

By limiting the number of superabsorbent particles per unit of volume, it has been found that it is possible to maintain a fibrous network with a pore structure which can transport liquid in the absorbent structure even after the structure has been subjected to a first wetting. It has also been found that, with a limited number of superabsorbent particles per unit of volume, it is essential that the average absorption capacity per superabsorbent particle is greater than 8.0 mg. The advantage of such an absorbent structure is that the risk of gel blocking decreases at the same time as it is possible to obtain a thin absorbent structure.

According to one embodiment, the average absorption capacity per superabsorbent particle in the absorbent layer is greater than 9.5 mg. The absorption capacity is measured using 0.9% by weight sodium chloride solution. Furthermore, the number of superabsorbent particles per $cm^3$ of the absorbent layer is smaller than 600.

According to another embodiment, the average absorption capacity per superabsorbent particle in the absorbent layer is greater than 14.0 mg. The absorption capacity is measured using 0.9% by weight sodium chloride solution. In such an embodiment, the number of superabsorbent particles per $cm^3$ of the absorbent layer is smaller than 450.

According to one embodiment, the superabsorbent particles have a particle size which is greater than 600 $\mu$m. The superabsorbent particles are preferably polyacrylate-based. In order to obtain a high absorption capacity, it is also possible to change the morphology of the superabsorbent particles. An example of superabsorbent particles with a changed morphological structure is microporous superabsorbent particles. A high absorption capacity can also be obtained by means of a special chemical composition of the superabsorbent particles.

The superabsorbent particles are surface cross-linked. A surface cross-linked superabsorbent is cross-linked in two different steps. First, the polymer is cross-linked so that a homogeneous cross-linked gel is formed. In cases where polymerization and cross-linking do not result in particles being formed simultaneously, particles are produced in a following process step. In another following process step, the formed particles are cross-linked in the second step, but then only partly. The additional cross-linking can be effected so that there is a higher cross-linker content next to the surface of the particle compared with the centre of the particle. In this way, a more firmly cross-linked particle shell is produced, which surrounds a particle core with a lower degree of cross-linking.

Superabsorbents with a low degree of cross-linking provide a high absorption capacity. However, a problem with such superabsorbents is that, in a swollen state, they are soft and sticky, which results in the risk of gel blocking in the absorbent structure already being high at a low superabsorbent material content. Superabsorbents with a high degree of cross-linking keep their shape better in a swollen state and do not stick to the same great extent either. However, a problem with a superabsorbent with a high degree of cross-linking is that it has a considerably lower absorption capacity. So, by surface cross-linking the superabsorbent, or alternatively creating a cross-linking gradient so that the particle surface is cross-linked more firmly than the inner particle core, a superabsorbent is obtained which both has high absorption capacity and essentially maintains its shape in a swollen state.

According to one embodiment of an absorbent structure according to the invention, the average distance between centres of the superabsorbent particles in the absorbent layer in a dry state is greater than 700 micrometres, more preferably greater than 1000 micrometres and even better greater than 1200 micrometres. The average centre-centre distance ($l_{cc}$) of the superabsorbent particles is obtained using the following equation:

$$l_{cc} = (1/n)^{1/3}$$

n = number of superabsorbent particles per unit of volume of material

According to another embodiment, the density of the absorbent layer is greater than 0.12 g/$cm^3$, more preferably greater than 0.17 g/$cm^3$ and even better greater than 0.25 g/$cm^3$. The absorbent layer can moreover comprise bonding means, such as bonding fibres for example. Examples of bonding fibres are synthetic fibres made of polyolefin. In order to function as bonding fibres, the fibres are heated to their melting point, the fibres being bonded to the material in the absorbent layer. Bonding fibres made of bicomponent fibres are common. If bicomponent fibres are used as bonding fibres, one component is melted while the other component is intact, that is to say does not melt but instead maintains the structure of the fibre.

The invention also relates to an absorbent article such as a diaper, an incontinence pad, or a sanitary towel, which comprises an absorbent structure according to any one of the embodiments described.

BRIEF DESCRIPTION OF THE FIGURES

Figure 1     shows a diaper according to the invention, seen from the side which is intended to lie against the wearer during use;

Figure 2     shows a cross section along the line II-II through the diaper shown in Figure 1;

Figure 3    shows a cross section of an alternative embodiment of an absorbent article according to the invention.

DETAILED DESCRIPTION OF THE FIGURES

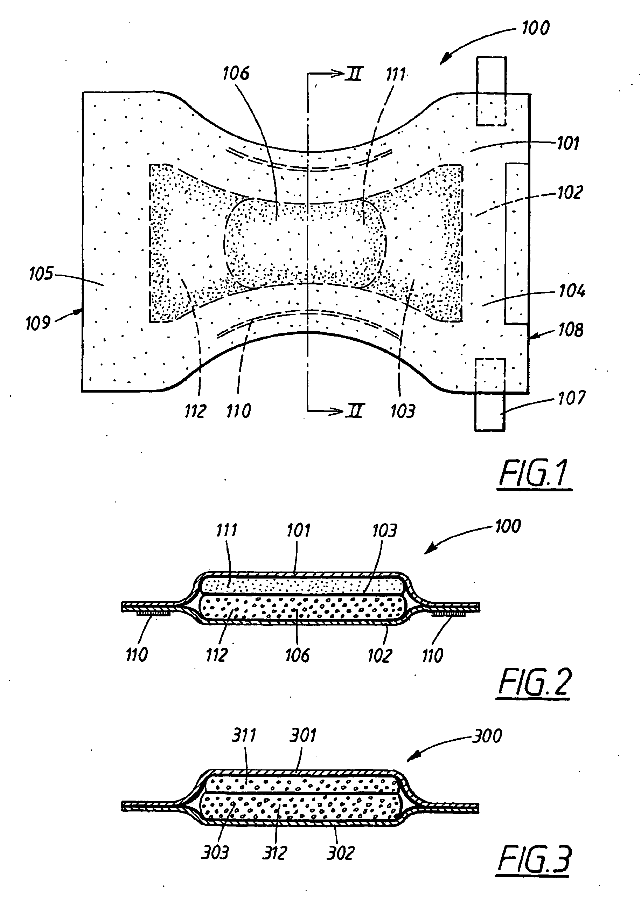

The diaper 100 shown in Figure 1 comprises a liquid-permeable surface layer 101, a backing layer 102, which is at least essentially liquid-impermeable, and an absorbent structure 103 enclosed between the liquid-permeable surface layer 101 and the backing layer 102.

The diaper is intended to surround the lower part of the abdomen of a wearer like a pair of absorbent underpants. To this end, it is shaped with a rear portion 104 and a front portion 105, and a narrower crotch portion 106 which is located between the front portion 105 and the rear portion 104 and is intended during use to be arranged in the crotch of the wearer between the legs of the latter. In order that it is possible for the diaper to be fastened together in the desired pants-shape, tape tabs 107 are arranged close to the rear waist edge 108 of the diaper. During use, the tape tabs 107 are fastened to the front portion 105 of the diaper, close to the front waist edge 109, so that the diaper is held together around the waist of the wearer. Other fastening devices are of course also possible, such as hook and loop fastening for example.

The diaper 100 according to Figure 1 also comprises pretensioned elastic means 110 which can consist of elastic bands, thread-covered elastic threads, elastic foam or another suitable material. For the sake of simplicity, the elastic means 110 have in Figure 1 been shown in the stretched state. As soon as stretching stops, however, they contract and form elastic leg-bands of the diaper.

The liquid-permeable surface layer 101 is, for example, a nonwoven material or a perforated film, or a laminate thereof. Examples of polymers from which the liquid-permeable surface layer 101 can be made are polyethylene, polypropylene, polyester or copolymers thereof. In order that the liquid-permeable surface layer 101 will allow the discharged body fluid to pass through rapidly, it is common for the surface layer to be surfactant-coated and/or perforated. Another suitable material for use as the liquid-permeable surface layer is a layer of continuous fibres which are interconnected in a spot, line or patch bonding pattern but are otherwise on the whole not bonded to one another. The backing layer 102 is, for example, a plastic film, which is preferably breathable, a hydrophobic nonwoven layer or a laminate thereof.

The absorbent structure 103 of the diaper 100 is constructed from an upper liquid-receiving layer 111 and a lower liquid-distribution and storage layer 112. The lower liquid-distribution and storage layer 112 has a greater extent in the plane of the article than the upper liquid-receiving layer 111. The upper receiving layer 111 is to be capable of rapidly receiving large quantities of liquid in a short time, that is to say have a high instantaneous liquid absorption capacity, while the lower distribution and storage layer 112 is to have a high wicking capacity and high storage capacity and to be capable of draining liquid from the receiving layer 111. The lower distribution and storage layer 112 in the absorbent structure 103 consists of an absorbent layer according to the invention. The lower liquid-distribution and storage layer 112 therefore also comprises superabsorbent particles in addition to cellulose fluff pulp. The average absorption capacity per superabsorbent particle in the liquid-distribution and storage layer 112 is greater than 8.0 mg sodium chloride solution. Furthermore, the number of superabsorbent particles per $cm^3$ of the liquid-spreading and storage layer 112 is smaller than 1100. According to one embodiment, the average absorption capacity per superabsorbent particle in the liquid-distribution and storage layer 112 is greater than 9.5 mg, and the number of superabsorbent particles per $cm^3$ of the liquid-distribution and storage layer is smaller than 600. According to another example, the average absorption capacity per superabsorbent particle in the liquid-distribution and storage layer 112 is greater than 14.0 mg, and the number of superabsorbent particles per $cm^3$ of the liquid-distribution and storage layer is smaller than 450. The absorption capacity is measured throughout using 0.9% by weight sodium chloride solution.

The average distance between centres of the superabsorbent particles in the liquid-distribution and storage layer 112 in a dry state is, for example, greater than 700 micrometres, preferably greater than 1000 micrometres and even better greater than 1200 micrometres. The density of the absorbent structure in the liquid-distribution and storage layer 112 is, for example, greater than 0.12 $g/cm^3$, preferably greater than 0.17 $g/cm^3$ and even better greater than 0.25 $g/cm^3$.

Suitable materials for use as the receiving layer 111 include, for example, an open nonwoven layer made of synthetic or natural fibres. A difference in properties between the liquid-distribution and storage layer 112 and the receiving layer 111 can be brought about by, for example, the liquid-distribution and storage layer 112 being compressed more firmly than the receiving layer 111. A firmly compressed fibrous structure with high density spreads the liquid better than a corresponding fibrous structure with lower density, which, because of its larger pore size, has a higher instantaneous liquid absorption capacity but lower wicking capacity. Differences in absorption properties between the two layers can also be brought about by means of different fibrous structures with different properties. Accordingly, cellulose fluff pulp produced in a conventional chemical way, chemical pulp (CP), has higher liquid-wicking capacity compared with pulp produced in a mechanical or chemithermomechanical way. Therefore, cellulose fluff pulp produced in a conventional chemical way, chemical pulp (CP), is an example of a suitable material for the liquid-distribution and storage layer 112, and pulp produced in a mechanical or chemithermomechanical way is an example of a material for the receiving layer 111. A fibrous structure containing chemically stiffened cellulose fibres also has a higher instantaneous liquid absorption capacity but lower spreading capacity than conventional chemical pulp and is therefore an example of a material for the receiving layer 111. Another suitable material for use as the receiving layer 111 is a superabsorbent foam, for example a polyacrylate-based foam. A polyacrylate-based foam is produced by a solution which consists of at least monomer, cross-linker, initiator and surfactant being saturated and pressurized with carbon dioxide in a vessel while being stirred. When the solution is removed from the vessel through a nozzle, the solution expands and a foamed structure is obtained. The foamed structure is then locked by polymerization and cross-linking being initiated by, for example, UV radiation. Finally, the material is compressed and dried. On wetting, such a superabsorbent foam expands greatly, which results in it being capable of receiving a large quantity of liquid in a short time. Such a receiving layer can consist of, for example, a continuous layer, which is positioned at least in the crotch portion of the article, or alternatively of a number of strips with hollow spaces between the strips. The receiving layer can also consist of a fibrous layer with superabsorbent particles or a superabsorbent coating bonded to the fibrous layer.

In order to reduce the occurrence of undesirable bacterial growth and problems with odour, the absorbent structure 103 and/or the liquid-permeable surface layer 101 can comprise bacteria-inhibiting and/or odour-inhibiting substances. An example of a bacteria-inhibiting and odour-inhibiting substance is a superabsorbent material which has a lower pH than a conventional superabsorbent. A superabsorbent material with a lower pH than a conventional superabsorbent has a lower degree of neutralization than a conventional superabsorbent, the degree of neutralization being, for example, between 20 and 60%. The superabsorbent particles according to the invention can be, for example, a superabsorbent with a degree of neutralization between 20 and 60%.

Figure 2 shows a cross section along the line II-II through the diaper 100 shown in Figure 1. The diaper 100 shown in Figure 2 therefore has a liquid-permeable surface layer 101, a backing layer 102, and an absorbent structure 103 enclosed between the liquid-permeable surface layer 101 and the backing layer 102.

The absorbent structure 103 of the diaper is constructed from an upper liquid-receiving layer 111 and a lower liquid-distribution and storage layer 112. The lower liquid-distribution and storage layer 112 in the absorbent structure 103 consists of an absorbent layer according to the invention. The liquid-distribution and storage layer 112 therefore also comprises superabsorbent particles in addition to cellulose fluff pulp. The average absorption capacity per superabsorbent particle in the liquid-spreading and storage layer 112 is greater than 8.0 mg sodium chloride solution, and the number of superabsorbent particles per $cm^3$ of the liquid-spreading and storage layer 112 is smaller than 1100.

Figure 3 shows a cross section of an alternative embodiment of an absorbent article according to the invention. The diaper 300 shown in Figure 3 is essentially constructed in the same way as the diaper in Figure 2. The diaper 300 therefore has a liquid-permeable surface layer 301, a backing layer 302, and an absorbent structure 303 enclosed between the liquid-permeable surface layer 301 and the backing layer 302.

The absorbent structure 303 of the diaper is constructed from an upper liquid-receiving layer 311 and a lower liquid-distribution and storage layer 312. Both the upper liquid-receiving layer 311 and the lower liquid-distribution and storage layer 312 in the absorbent structure 303 consist of absorbent layers according to the invention. The upper liquid-receiving layer 311 and the lower liquid-distribution and storage layer 312 therefore consist of fibrous structures which comprise superabsorbent particles, the average absorption capacity per superabsorbent particle in both layers 311, 312 being greater than 8.0 mg sodium chloride solution. Furthermore, the number of superabsorbent particles per $cm^3$ of the absorbent structure in both layers is smaller than 1100. In the absorbent structure 303, both the upper liquid-receiving layer 311 and the lower liquid-distribution and storage layer 312 therefore consist of absorbent layers according to the invention.

The invention is of course not limited to the illustrative embodiments above. The invention therefore also comprises incontinence pads, pant diapers, sanitary towels, and panty liners. The invention also includes belt-supported diapers.

It is furthermore possible, for example, for the whole of the absorbent structure to consist of only one absorbent layer, in which case the whole absorbent structure consists of an absorbent layer according to the invention. According to another example, the absorbent structure can consist of a multilayer structure where the upper liquid-receiving layer consists of an absorbent layer according to the invention. The liquid-receiving layer therefore comprises fibres and superabsorbent particles, the average absorption capacity for the superabsorbent particles in the liquid-receiving layer being greater than 8.0 mg sodium chloride solution (% by weight of sodium chloride is 0.9%), and the number of superabsorbent particles per $cm^3$ of the liquid-receiving layer being smaller than 1100. A cellulose fluff pulp with superabsorbent material of conventional type, for example, is used as the liquid-distribution and storage layer. It is also possible for the liquid-distribution and storage layer to consist of several different layers, at least one of the layers containing superabsorbent material and, for example, one layer consisting of pure pulp in order to obtain good liquid distribution. Different layers can moreover have a difference in concentration of superabsorbent material, a liquid-distribution and storage layer with a gradually increasing/decreasing content of superabsorbent material being obtained. It is also possible for the liquid-distribution and storage layer to consist of or comprise a layer made of a superabsorbent foam.

It is furthermore also possible for the absorbent structure to include one or more tissue layers or types of material or component other than those described above. The design of the layers can also vary. For example, one or more layers in the absorbent structure-can have cut-outs, that is to say cavities. The cut-outs extend, for example, in the longitudinal direction of the absorption structure. It is of course also possible to have other physical designs of cut-out.

Example 1 - Determining volume and density of the absorbent layer.

When measuring the volume ($cm^3$) of the absorbent layer in an absorbent article, the absorbent layer is separated from the rest of the material in the article. If the absorbent structure has several different absorbent layers with mutually different properties, the various absorbent layers are also separated from one another, after which volume and density are measured for each absorbent layer.

The absorbent layer is then weighed, and the thickness of the absorbent layer is measured. When measuring the thickness, use is made of a thickness gauge which has a circular foot with a diameter of 80 mm. The foot is to exert a pressure of 0.5 kPa on the absorbent layer. The thickness is measured at five different points, which are distributed uniformly over the surface of the absorbent layer. The average value from these five measuring points represents the thickness of the absorbent layer in the volume calculation. The area of the absorbent layer is then measured, the volume being obtained by multiplying the thickness by the area. The density of the absorbent layer is then obtained by dividing the weight of the absorbent layer by the volume.

Example 2 - Determining the number of superabsorbent particles per unit of volume and measuring the absorption capacity of the superabsorbent particles.

The example is based on an absorbent layer which contains the superabsorbent particles. In this connection, it is also described how the superabsorbent particles are to be separated from the pulp structure. It is of utmost importance than no material is lost in the handling described below. Ensure therefore that necessary measures are taken to avoid loss.

The absorbent layer is first separated from the rest of the material in the article. The superabsorbent particles are then separated from the fluff pulp in the absorbent layer by finely dividing the layer, that is to say tearing it into small pieces, and then shaking the superabsorbent particles out of the pulp structure. It is also possible to use an apparatus for separating the superabsorbent particles from the pulp structure. If an apparatus is used for separating the superabsorbent particles from the pulp structure, however, it is a condition that the superabsorbent particles are not damaged mechanically. The moisture content of the superabsorbent particles is to be less than 5.0%. All indications in the present invention relate to superabsorbent particles with a moisture content of less than 5.0%. The moisture content is determined according to the method ISO 17190-4 "Determination of moisture content by mass loss upon heating". If the moisture content exceeds 5.0%, the superabsorbent is dried at 60°C until the moisture content is less than 5.0%.

Particles with a diameter smaller than 150 $\mu$m are then separated. All indications of the number of superabsorbent particles in the present invention relate to particles with a diameter of 150 $\mu$m or greater. Particles with a diameter smaller than 150 $\mu$m are therefore not included in the expression "superabsorbent particles" according to the invention. In order to separate particles smaller than 150 $\mu$m, use is made of apparatus described in ISO 17190-3 "Determination of particle size distribution by sieve fractionation". Particles smaller than 150 $\mu$m are sieved out. The remaining particles are then weighed. This weight therefore constitutes the total weight of the superabsorbent particles.

In order to calculate the number of superabsorbent particles per unit of volume, the superabsorbent particles are divided up into smaller portions. To divide the superabsorbent particles up into smaller portions, use is made of a "Rotary Sample Divider - laborette 27" from Fritsch GmbH Laborgerätebau or similar apparatus. Each portion is assumed to have a representative particle size distribution. Three of these portions are then weighed, and the number of particles in these three portions is counted manually. Each portion weighed 0.1 gram, so altogether the number of superabsorbent particles in 0.3 gram was counted. The weight of the samples is to be +/- 10% of the given values. The measuring accuracy is to be +/- 0.005 gram. The average weight of the individual superabsorbent particles is then calculated by dividing the weight of the sample (roughly 0.3 gram) by the number of manually counted particles. By then dividing the total weight of the superabsorbent particles by the average weight of the superabsorbent particles, the total number of superabsorbent particles in the absorbent layer is obtained. In order finally to obtain the number of superabsorbent particles per $cm^3$ of the absorbent layer, the total number of superabsorbent particles is divided by the volume of the absorbent layer.

The absorption capacity of the superabsorbent particles is then measured according to ISO 17190-6 "Gravimetric determination of fluid retention after centrifugation". The absorption capacity is measured on three other portions. All portions have a representative particle size distribution, and the absorption capacity per particle can therefore be calculated by dividing the measured absorption capacity by the number of particles previously counted manually.

Liquid used for measurement is 0.9% by weight sodium chloride solution.

Superabsorbent materials tested are three different size fractions of particulate polyacrylate-based superabsorbent from BASF with the designation Hysorb C 7100 and two different size fractions of particulate polyacrylate-based superabsorbent from Dow with the designation Drytech S230R. The average particle size of the superabsorbent particles from BASF with the designation Hysorb C 7100 was in the first case a normal particle-size distribution, that is to say the measurement was performed on the whole particle fraction in the commercially available grade; in the second case the particle size was between 600 $\mu$m and 710 $\mu$m, and in the third case the average particle size was between 710 $\mu$m and 850 $\mu$m. The average particle size of the superabsorbent particles from Dow with the designation Drytech S230R was in one case a normal particle size distribution, that is to say the measurement was performed on the whole particle fraction in the commercially available grade, and the average particle size in the other case was greater than 600 $\mu$m.

The superabsorbent with the designation Hysorb C 7100 with a normal particle size distribution is called A below.

The superabsorbent with the designation Hysorb C 7100 with a particle size between 600 $\mu$m and 710 $\mu$m is called B below.

The superabsorbent with the designation Hysorb C 7100 with a particle size between 710 $\mu$m and 850 $\mu$m is called C below.

The superabsorbent with the designation Drytech S230R with a normal particle size distribution is called D below.

The superabsorbent Drytech S230R with a particle size greater than 600 $\mu$m is called E below.

Result

| Superabsorbent | Abs. cap. (g/g) | Abs. cap/particle (mg/particle) |
| --- | --- | --- |
| A | 35.6 | 1.4 |
| B | 38.0 | 7.4 |
| C | 37.2 | 9.5 |
| D | 33.6 | 1.7 |
| E | 36.5 | 8.4 |

It can be seen from the result that the average absorption capacity per particle for superabsorbent C and E is greater than 8.0 mg sodium chloride solution, while the average absorption capacity per particle for superabsorbent A, B and D is less than 8.0 mg sodium chloride solution.

Example 3 - Measuring admission time in absorbent structure.

Measurement of admission time on a first, a second, a third and a fourth measurement was performed for five different absorbent structures. The absorbent structures contained 50% by weight superabsorbent and 50% by weight chemical fluff pulp. The chemical fluff pulp was manufactured by Weyerhauser and is called NB 416.

Superabsorbent material in absorbent structure 1 was superabsorbent A, that is to say Hysorb C 7100 with a normal particle size distribution.

Superabsorbent material in absorbent structure 2 was superabsorbent B, that is to say Hysorb C 7100 with a particle size between 600 $\mu$m and 710 $\mu$m.

Superabsorbent material in absorbent structure 3 was superabsorbent C, that is to say Hysorb C 7100 with a particle size between 710 $\mu$m and 850 $\mu$m.

Superabsorbent material in absorbent structure 4 was superabsorbent D, that is to say Drytech S230R with a normal particle size distribution.

Superabsorbent material in absorbent structure 5 was superabsorbent E, that is to say Drytech S230R with a particle size greater than 600 $\mu$m.

The absorbent structures 1, 2 and 3 had a density which was 0.25 g/cm$^3$, a weight per unit area which was 600 g/m$^2$ and an area which was 10x28 cm.

The absorbent structures 4 and 5 had a density which was 0.25 g/cm$^3$, a weight per unit area which was 600 g/m$^2$ and an area which was 10x40 cm.

For measurement, the absorbent structure was placed on a foam mattress of the tempur type. The absorbent structure was then subjected to a load of 0.64 kPa and four doses of 80 ml each of sodium chloride solution (0.9% by weight) were added. The time between the liquid doses was 10 minutes. The time for the liquid to be admitted into the absorbent structure was measured. The admission time was measured in seconds.

Result

|  | Abs. struct. 1 (s) | Abs. struct. 2 (s) | Abs. struct. 3 (s) |
|---|---|---|---|
| 1st wetting | 115 | 103 | 110 |
| 2nd wetting | 210 | 149 | 146 |
| 3rd wetting | 312 | 230 | 217 |
| 4th wetting | 354 | 275 | 259 |

|  | Abs. struct. 4 (s) | Abs. struct. 5 (s) |
|---|---|---|
| 1st wetting | 67 | 83 |
| 2nd wetting | 80 | 69 |
| 3rd wetting | 122 | 109 |
| 4th wetting | 160 | 132 |

The result shows that of the absorbent structures which contained superabsorbent Hysorb C 7100, that is to say absorbent structures 1-3, absorbent structure 3 has the fastest admission time on repeated wetting. Absorbent structure 3 contained superabsorbent particles with an average absorption capacity which is greater than 8.0 mg.

Of the absorbent structures which contained Drytech S230R, that is to say absorbent structures 4-5, absorbent structure 5 has the fastest admission time on repeated wetting. Absorbent structure 5 contained superabsorbent particles with an average absorption capacity which is greater than 8.0 mg, while absorbent structure 4 contained superabsorbent particles with an average absorption capacity which is lower than 8.0 mg.

Example 4 - Measuring liquid distribution in absorbent structure.

Measurement of liquid distribution after a first, a second, a third and a fourth wetting was performed for absorbent structure 4 and absorbent structure 5. The liquid distribution was measured after each wetting immediately before the next liquid dose was added. The liquid distribution was measured in cm.

Result

|  | Abs. struct. 4 (cm) | Abs. struct. 5 (cm) |
|---|---|---|
| 1st wetting | 20 | 25 |
| 2nd wetting | 22 | 26 |
| 3rd wetting | 29 | 34 |
| 4th wetting | 34 | 39 |

The result shows that absorbent structure 5 which contained superabsorbent particles with an average absorption capacity which is greater than 8.0 mg spreads the liquid further than absorbent structure 4 which contained superabsorbent particles with an average absorption capacity which is lower than 8.0 mg.

Example 5 - Measuring rewet.

Measurement of rewet after the fourth wetting was performed for absorbent structure 4 and absorbent structure 5. Measurement of rewet was started 10 minutes after the fourth dose of liquid had been applied. After 10 minutes, 15 pieces of filter paper and a weight (5 kPa) were placed on the wetting point of the absorbent structure. After 15 seconds, the weight was removed, and the bundle of filter paper was weighed. The rewet was calculated by subtracting the dry weight of the filter paper from the wet weight. The rewet was measured in grams of sodium chloride solution (0.9% by weight).

Result

Absorbent structure 4: 9.5 grams
Absorbent structure 5: 7.9 grams
The result shows that absorbent structure 5 has lower rewet than absorbent structure 4. The result therefore shows that the absorbent structure which contained superabsorbent particles with an average absorption capacity which is greater than 8.0 mg has lower rewet than the absorbent structure which contained superabsorbent particles with an average absorption capacity which is lower than 8.0 mg.

**Claims**

1. Absorbent structure (103; 303) for use in an absorbent article, which absorbent structure (103; 303) has at least one absorbent layer (112; 311; 312) comprising fluff pulp and superabsorbent particles, **characterized in that** the average absorption capacity per superabsorbent particle in the absorbent layer (112; 311; 312) is greater than 8.0 mg sodium chloride solution, **that** the number of superabsorbent particles per $cm^3$ of the absorbent layer (112; 311; 312) is smaller than 1100, and **in that** the superabsorbent particles are surface cross-linked.

2. Absorbent structure (103; 303) according to Claim 1, **characterized in that** the average absorption capacity per superabsorbent particle in the absorbent layer (112; 311; 312) is greater than 9.5 mg sodium chloride solution and **in that** the number of superabsorbent particles per $cm^3$ of the absorbent layer is smaller than 600.

3. Absorbent structure (103; 303) according to Claim 2, **characterized in that** the average absorption capacity per superabsorbent particle in the absorbent layer (112; 311; 312) is greater than 14.0 mg sodium chloride solution and **in that** the number of superabsorbent particles per $cm^3$ of the absorbent layer (112; 311; 312) is smaller than 450.

4. Absorbent structure (103; 303) according to any one of Claims 1-3, **characterized in that** the superabsorbent particles have a particle size which is greater than 600 $\mu$m.

5. Absorbent structure (103; 303) according to any one of Claims 1-4, **characterized in that** the average distance between centres of the superabsorbent particles in the absorbent layer (112; 311; 312) in a dry state is greater than 700 $\mu$m.

6. Absorbent structure (103; 303) according to Claim 5, **characterized in that** the average distance between centres of the superabsorbent particles in the absorbent layer (112; 311; 312) in a dry state is greater than 1000 $\mu$m.

7. Absorbent structure (103; 303) according to Claim 6, **characterized in that** the average distance between centres of the superabsorbent particles in the absorbent layer (112; 311; 312) in a dry state is greater than 1200 $\mu$m.

8. Absorbent structure (103; 303) according to any one of Claims 1-7, **characterized in that** the density of the absorbent layer (112; 311; 312) in a dry state is greater than 0.12 $g/cm^3$.

9. Absorbent structure (103; 303) according to Claim 8, **characterized in that** the density of the absorbent layer (112; 311; 312) in a dry state is greater than 0.17 $g/cm^3$.

10. Absorbent structure (103; 303) according to Claim 9, **characterized in that** the density of the absorbent layer (112; 311; 312) in a dry state is greater than 0.25 $g/cm^3$.

11. Absorbent structure (103; 303) according to any one of the preceding claims, **characterized in that** the absorbent layer (112; 311; 312) comprises bonding means.

12. Absorbent article such as a diaper (100; 300), an incontinence pad, or a sanitary towel, **characterized in that** it comprises an absorbent structure (103; 303) according to any one of Claims 1-11.

**Patentansprüche**

1. Absorbierende Struktur (103, 303) für die Verwendung in einem absorbierenden Gegenstand, wobei die absorbierende Struktur (103, 303) mindestens eine absorbierende Schicht (112, 311, 312) aufweist, die Fluffzellstoff und superabsorbierende Partikel aufweist, **dadurch gekennzeichnet, daß** die durchschnittliche Absorptionskapazität pro superabsorbierendem Partikel in der absorbierenden Schicht (112, 311, 312) größer ist als 8,0 mg Natriumchloridlösung, daß die Zahl superabsorbierender Partikel pro cm$^3$ der absorbierenden Schicht (112, 311, 312) kleiner als 1.100 ist, und daß die superabsorbierenden Partikel oberflächenvernetzt sind.

2. Absorbierende Struktur (103, 303) gemäß Anspruch 1, **dadurch gekennzeichnet, daß** die durchschnittliche Absorptionskapazität pro superabsorbierendem Partikel in der absorbierenden Schicht (112, 311, 312) größer ist als 9,5 mg Natriumchloridlösung und daß die Zahl superabsorbierender Partikel pro cm$^3$ der absorbierenden Schicht kleiner als 600 ist.

3. Absorbierende Struktur (103, 303) gemäß Anspruch 2, **dadurch gekennzeichnet, daß** die durchschnittliche Absorptionskapazität pro superabsorbierendem Partikel in der absorbierenden Schicht (112, 311, 312) größer ist als 14,0 mg Natriumchloridlösung und daß die Zahl superabsorbierender Partikel pro cm$^3$ der absorbierenden Schicht (112, 311, 312) kleiner als 450 ist.

4. Absorbierende Struktur (103, 303) gemäß mindestens einem der Ansprüche 1 bis 3, **dadurch gekennzeichnet, daß** die superabsorbierenden Partikel eine Partikelgröße haben, die größer als 600 μm ist.

5. Absorbierende Struktur (103, 303) gemäß mindestens einem der Ansprüche 1 bis 4, **dadurch gekennzeichnet, daß** der mittlere Abstand zwischen Zentren der superabsorbierenden Partikel in der absorbierenden Schicht (112, 311, 312) in trockenem Zustand größer als 700 μm ist.

6. Absorbierende Struktur (103, 303) gemäß Anspruch 5, **dadurch gekennzeichnet, daß** der mittlere Abstand zwischen Zentren der superabsorbierenden Partikel in der absorbierenden Schicht (112, 311, 312) in trockenem Zustand größer als 1.000 μm ist.

7. Absorbierende Struktur (103, 303) gemäß Anspruch 6, **dadurch gekennzeichnet, daß** der mittlere Abstand zwischen Zentren der superabsorbierenden Partikel in der absorbierenden Schicht (112, 311, 312) in trockenem Zustand größer als 1.200 μm ist.

8. Absorbierende Struktur (103, 303) gemäß mindestens einem der Ansprüche 1 bis 7, **dadurch gekennzeichnet, daß** die Dichte der absorbierenden Schicht (112, 311, 312) in trockenem Zustand größer als 0,12 g/cm$^3$ ist.

9. Absorbierende Struktur (103, 303) gemäß Anspruch 8, **dadurch gekennzeichnet, daß** die Dichte der absorbierenden Schicht (112, 311, 312) in trockenem Zustand größer als 0,172 g/cm$^3$ ist.

10. Absorbierende Struktur (103, 303) gemäß Anspruch 9, **dadurch gekennzeichnet, daß** die Dichte der absorbierenden Schicht (112, 311, 312) in trockenem Zustand größer als 0,25 g/cm$^3$ ist.

11. Absorbierende Struktur (103, 303) gemäß mindestens einem der vorhergehenden Ansprüche, **dadurch gekennzeichnet, daß** die absorbierende Schicht (112, 311, 312) Bindungsmittel umfaßt.

12. Absorbierender Artikel wie eine Windel (100, 300), ein Inkontinenzkissen oder eine Damenbinde, **dadurch gekennzeichnet, daß** er eine absorbierende Struktur (103, 303) gemäß mindestens einem der Ansprüche 1 bis 11 umfaßt.

**Revendications**

1. Structure absorbante (103 ; 303) pour une utilisation dans un article absorbant, cette structure absorbante (103 ; 303) ayant au moins une couche absorbante (112 ; 311 ; 312) comprenant de la pâte en flocons et des particules superabsorbantes, **caractérisé en ce que** la capacité d'absorption moyenne par particule superabsorbante dans la couche absorbante (112 ; 311 ; 312) est supérieure à 8,0 mg d'une solution de chlorure de sodium, **en ce que** le nombre de particules superabsorbantes par cm$^3$ de la couche absorbante (112 ; 311 ; 312) est inférieur à 1 100, et **en ce que** les particules superabsorbantes sont réticulées en surface.

**2.** Structure absorbante (103 ; 303) selon la revendication 1, **caractérisée en ce que** la capacité d'absorption moyenne par particule superabsorbante dans la couche absorbante (112 ; 311 ; 312) est supérieure à 9,5 mg d'une solution de chlorure de sodium, et **en ce que** le nombre de particules superabsorbantes par $cm^3$ de la couche absorbante est inférieur à 600.

**3.** Structure absorbante (103 ; 303) selon la revendication 2, **caractérisée en ce que** la capacité d'absorption moyenne par particule superabsorbante dans la couche absorbante (112 ; 311 ; 312) est supérieure à 14,0 mg d'une solution de chlorure de sodium, et **en ce que** le nombre de particules superabsorbantes par $cm^3$ de la couche absorbante (112 ; 311 ; 312) est inférieur à 450.

**4.** Structure absorbante (103 ; 303) selon l'une quelconque des revendications 1 à 3, **caractérisée en ce que** les particules superabsorbantes ont une taille particulaire qui est supérieure à 600 $\mu$m.

**5.** Structure absorbante (103 ; 303) selon l'une quelconque des revendications 1 à 4, **caractérisée en ce que** la distance moyenne entre les centres des particules superabsorbantes dans la couche absorbante (112 ; 311 ; 312) à l'état sec est supérieure à 700 $\mu$m.

**6.** Structure absorbante (103 ; 303) selon la revendication 5, **caractérisée en ce que** la distance moyenne entre les centres des particules superabsorbantes dans la couche absorbante (112 ; 311; 312) à l'état sec est supérieure à 1 000 $\mu$m.

**7.** Structure absorbante (103 ; 303) selon la revendication 6, **caractérisée en ce que** la distance moyenne entre les centres des particules superabsorbantes dans la couche absorbante (112 ; 311; 312) à l'état sec est supérieure à 1 200 $\mu$m.

**8.** Structure absorbante (103 ; 303) selon l'une quelconque des revendications 1 à 7, **caractérisée en ce que** la masse volumique de la couche absorbante (112 ; 311 ; 312) à l'état sec est supérieure à 0,12 $g/cm^3$.

**9.** Structure absorbante (103 ; 303) selon la revendication 8, **caractérisée en ce que** la masse volumique de la couche absorbante (112 ; 311 ; 312) à l'état sec est supérieure à 0,17 $g/cm^3$.

**10.** Structure absorbante (103; 303) selon la revendication 9, **caractérisée en ce que** la masse volumique de la couche absorbante (112 ; 311 ; 312) à l'état sec est supérieure à 0,25 $g/cm^3$.

**11.** Structure absorbante (103 ; 303) selon l'une quelconque des revendications précédentes, **caractérisée en ce que** la couche absorbante (112 ; 311 ; 312) comprend des moyens de liaison.

**12.** Article absorbant, tel qu'une couche (100 ; 300), un tampon d'incontinence ou une serviette hygiénique, **caractérisé en ce qu'**il comprend une structure absorbante (103 ; 303) selon l'une quelconque des revendications 1 à 11.

_FIG.1_

_FIG.2_

_FIG.3_

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005032443 A

- EP 0212618 A1